# FASCICULE DE BREVET EUROPEEN

(11) **EP 0 673 245 B1**
(45) Date de publication et mention de la délivrance du brevet: **24.09.1997**
(21) Numéro de dépôt: 94902008.5
(22) Date de dépôt: 09.12.1993
(51) Int. Cl.: A61K 31/19, A61K 9/00

(54) **COMPOSITION PHARMACEUTIQUE EFFERVESCENTE CONTENANT DE L'IBUPROFENE ET SON PROCEDE DE PREPARATION**
BRAUSEMISCHUNGEN ENTHALTEND IBUPROFEN UND VERFAHREN
EFFERVESCENT PHARMACEUTICAL COMPOSITION CONTAINING IBUPROFEN AND METHOD FOR PREPARING SAME

(30) Priorité: 09.12.1992 FR 9214851
(43) Date de publication de la demande: 27.09.1995
(73) Titulaire: LABORATOIRES UPSA, 47000 Agen (FR)
(72) Inventeur: BRU-MAGNIEZ, Nicole, F-75016 Paris (FR); CORDOLIANI, Jean-François, F-47390 Layrac (FR); THAUVIN, Gérard, F-47000 Agen (FR); DROUIN, Jehan-Yves, F-91370 Verrières-le-Buisson (FR)
(74) Mandataire: Hubert, Philippe
(86) Numéro de dépôt international: FR9301216
(87) Numéro de publication internationale: WO9413279

(56) Documents cités:
- EP-A- 0 351 353
- EP-A- 0 380 367

## Description

La présente invention a pour objet une nouvelle composition pharmaceutique effervescente contenant à titre d'ingrédient actif de l'ibuprofène et son procédé de préparation.

L'acide 2-(4-isobutylphenyl) propionique, connu sous le nom d'ibuprofène est un médicament bien toléré, doté d'activités analgésique antipyrétique et anti-inflammatoire (Merk Index, XI édition, n° 4812).

Diverses solutions ont donc été proposées, dans l'état de la technique, pour préparer des médicaments à base d'ibuprofène, sous forme de poudres ou de comprimés effervescents.

C'est ainsi que dans le document FR- 2590 893, il est proposé une composition, destinée à la préparation d'un comprimé ou granulé effervescent, comprenant en partie en poids :
9 à 17 % d'ibuprofène
17 à 33 % d'arginine
20 à 35 % de bicarbonate de sodium ou de potassium
25 à 40 % de bitartrate de sodium
le tout formant 100 %.

L'utilisation de bitartrate de sodium et d'arginine est présentée dans ce document antérieur comme obligatoire pour obtenir une bonne dissolution de l'ibuprofène.

Dans le document EP-A-0228164, il est proposé une composition destinée à la préparation d'une poudre ou d'un comprimé effervescent comprenant de l'ibuprofène ou l'un de ses sels pharmaceutiquement acceptables, un couple effervescent susceptible de produire du gaz carbonique en présence d'eau, un agent tensio actif et un polymère hydrophile insoluble.

Selon ce document antérieur, l'ibuprofène est de préférence enrobé par le polymère hydrophile insoluble (constitué avantageusement par de la cellulose microcristalline) et il est indiqué qu'en absence d'un tel polymère l'ibuprofène se dépose sur les parois du récipient dans lequel la composition effervescente est préparée.

Dans le document EP-A-351353, il est proposé une autre composition destinée à la préparation de poudres ou de comprimés effervescents comprenant :
200 à 800 mg d'ibuprofène,
221,3 à 885,2 mg du sel de sodium de l'ibuprofène
2100 à 8402 g de bicarbonate de sodium
0,450 à 1800 g d'acide citrique.

Selon ce document antérieur, l'utilisation de proportions spécifiques d'ibuprofène, de bicarbonate de sodium et d'acide citrique ainsi que l'utilisation d'un procédé spécifique de granulation (granulation dans un granulateur à lit fluidisé) permettent d'obtenir des comprimés effervescents d'ibuprofène complètement soluble sans goût amer, ne causant pas d'irritation de la gorge lors de leur utilisation.

La présente invention a pour but de fournir une nouvelle composition destinée à la préparation de poudres ou de comprimés effervescents d'ibuprofène à stabilité améliorée.

Il a en effet été constaté que l'ibuprofène se dégrade en présence et au contact des carbonates alcalins.

Cette interaction est surtout gênante pour la réalisation de comprimés effervescents qui nécessite :
- d'une part, la présence de fortes quantités de carbonates alcalins pour assurer une désagrégation rapide du comprimé;
- d'autre part, une étape de compression qui renforce le contact entre l'ibuprofène et les carbonates alcalins.

Ainsi, selon un premier aspect, la présente invention a pour objet une composition pharmaceutique destinée à la préparation de poudres ou de comprimés effervescents contenant une quantité efficace d'ibuprofène ou de l'un de ses sels pharmaceutiquement acceptables à titre d'ingrédient actif ; un système effervescent pharmaceutiquement acceptable comprenant au moins un carbonate alcalin et au moins un acide organique, caractérisée en ce qu'elle comprend au moins un agent antioxydant pharmaceutiquement acceptable, en une quantité suffisante pour stabiliser l'ibuprofène.

Ainsi, la présente invention est fondée sur la découverte surprenante du fait que la stabilité de l'ibuprofène, ou de ses sels pharmaceutiquement acceptables, au contact des carbonates alcalins peut être considérablement améliorée par un traitement préalable de l'ibuprofène ou de ses sels pharmaceutiquement acceptables par un agent antioxydant.

Dans le cadre de la présente description et des revendications, on entend désigner par poudres tout mélange de composants granulé ou non, destiné à être mis en solution et/ou en suspension dans l'eau, ou encore à être ingéré directement ou par tout autre moyen approprié comme par exemple en mélange avec un produit alimentaire.

Il est à noter que la présente invention s'applique préférentiellement à l'ibuprofène ainsi qu'à ses sels insolubles tels que notamment le sel de calcium, mais son principe peut être également utilisé pour la préparation de poudres ou de comprimés incorporant des sels solubles de l'ibuprofène.

Un carbonate alcalin susceptible d'être utilisé dans le cadre de la présente invention est par exemple le bicarbonate de sodium, le bicarbonate de potassium, le carboxylysine, le carbonate de calcium, le carbonate de sodium, le carbonate de potassium, ainsi que des mélanges de ces composés.

Avantageusement, on utilisera le bicarbonate de sodium.

Les acides organiques susceptibles d'être utilisés dans le cadre de la présente invention sont des composés capables de réagir avec les carbonates alcalins pour provoquer un dégagement de dioxyde de carbone, quand ils sont mis en contact avec suffisamment d'eau. Des acides convenables sont par exemple l'acide citrique, l'acide fumarique, l'acide adipique, l'acide tartrique ainsi que des mélange de ces composés.

L'acide préféré est l'acide citrique.

Selon un mode de réalisation particulier, on utilisera des quantités de carbonate alcalin et d'acide organique suffisantes pour obtenir un pH inférieur à environ 8 en présence d'eau, et de préférence un pH compris entre 4 et 5.

Dans le mode de réalisation préféré où le système effervescent est constitué d'un mélange de bicarbonate de sodium et d'acide citrique, les proportions en poids relatives de ces composés au sein du système effervescent varient en fonction du pH désiré après dissolution, ce qui est compréhensible pour l'homme de l'art, par exemple pour un pH d'environ 3 à environ 7 le rapport varie d'environ 20/80 à environ 80/20.

Selon une caractéristique particulière de l'invention, cette composition comprend en outre une quantité efficace d'au moins un déshydratant interne.

Il a en effet été découvert que la présence d'un déshydratant interne au sein de la composition renforce encore de façon notable la stabilité de l'ibuprofène et de ses sels pharmaceutiquement acceptables, vis-à-vis des carbonates alcalins, comme il sera expliqué plus loin.

Selon une autre caractéristique particulière, cette composition comprend en outre un diluant pharmaceutiquement acceptable.

Ce diluant joue également un rôle favorable dans la stabilisation de l'ibuprofène et des ses sels pharmaceutiquement acceptables.

Selon une caractéristique particulière de l'invention, l'agent antioxydant précité est choisi parmi l'alpha-tocophérol, le gamma-tocophérol, le delta-tocophérol, les extraits d'origine naturelle riches en tocophérol, l'acide L-ascorbique et ses sels de sodium ou de calcium, l'acide palmityl DL ascorbique, le gallate de propyle, le gallate d'octyle, le gallate de dodécyle, le gallate de butyl-hydroxy anisole (BHA) et le gallate de butyl-hydroxy toluène (BHT).

Selon un mode de réalisation actuellement préféré, l'agent antioxydant sera l'alpha-tocophérol.

Selon une autre caractéristique particulière de l'invention, le déshydratant interne est le citrate de magnésium.

Le déshydratant interne a pour fonction de piéger les traces d'eau susceptibles d'apparaître éventuellement dans le comprimé. Il a en effet été constaté que lors de son contact avec les carbonates alcalins, l'ibuprofène se dégrade en générant des molécules d'eau qui favorisent encore le processus de dégradation.

Selon une autre caractéristique particulière de l'invention, le diluant est le lactose.

Cependant tout autre diluant pharmaceutiquement acceptable pourraît être utilisé, en particulier le sucrose. Le citrate de magnésium peut également être utilisé dans cette fonction.

Les quantités respectives d'agent antioxydant, de déshydratant interne et de diluant pourront être facilement déterminées par un homme de métier et dépendent bien entendu de la forme pharmaceutique finale souhaitée.

D'une façon générale, une composition conforme à la présente invention destinée à la préparation de comprimés, peut contenir, exprimé en parties en poids pour 100 parties d'ibuprofène ou de l'un de ses sels pharmaceutiquement acceptables :
- de 120 à 900 et de préférence de 245 à 330 parties en poids d'au moins un carbonate alcalin;
- de 150 à 1100, et de préférence de 390 à 475 parties en poids d'au moins un acide organique:
- de 21.10⁻³ à 84.10⁻³ d'antioxydant, dans le cas de l'alpha-tocophérol;
et éventuellement :
- de 400 à 1000, et de préférence de 500 à 800 parties en poids d'au moins un diluant pharmaceutiquement acceptable ; et
- de 10 à 150, et de préférence de 20 à 100 parties en poids d'un déshydratant interne, dans le cas du citrate de magnesium.

Une composition pour comprimé, préférée, comprend, exprimé en parties en poids pour 100 parties d'ibuprofène :
- 270 parties d'au moins un carbonate alcalin, de préférence du bicarbonate de sodium;
- 450 parties en poids d'au moins un acide organique, de préférence l'acide citrique;
- 42.10⁻³ parties en poids d'alpha-tocophérol;
- 680 parties en poids d'un diluant, de préférence le lactose;
- 25 parties en poids de citrate de magnésium.

D'une façon générale, une composition conforme à la présente invention, destinée à la préparation de poudres, peut contenir, exprimé en parties en poids pour 100 parties d'ibuprofène ou de l'un de ses sels pharmaceutiquement acceptables :
- de 25 à 1200 et de préférence de 270 à 585 parties en poids d'au moins un carbonate alcalin;
- de 40 à 1125, et de préférence de 315 à 675 parties en poids d'au moins un acide organique;
- de 21.10⁻³ à 84.10⁻³ d'antioxydant, dans le cas de l'alpha-tocophérol;
et éventuellement :
- de 90 à 6000, et de préférence de 20 à 2100 parties en poids d'au moins un diluant pharmaceutiquement acceptable ; et
- de 0 à 100, et de préférence de 20 à 50 parties en poids d'un déshydratant interne, dans le cas du citrate de magnésium.

Une composition pour sachet, actuellement préférée, comprend, exprimé en parties en poids pour 100 parties d'ibuprofène ;
- 75 parties d'au moins un carbonate alcalin, de préférence du bicarbonate de sodium ;
- 225 parties en poids d'au moins un acide organique, de préférence l'acide citrique ;
- 42.10⁻³ parties en poids d'alpha-tocophérol ;
- 175 parties en poids d'un diluant, de préférence le lactose;
- 25 parties en poids de citrate de magnésium.

Selon un deuxième aspect, la présente invention a pour object une préparation pharmaceutique, sous la forme d'une poudre ou d'un comprimé effervescent, caractérisée en ce qu'elle contient une composition telle que définie précédemment éventuellement associée à au un moins un additif usuel choisi parmi les édulcorants, les aromatisants, les colorants et les lubrifiants.

Le choix de ces additifs et de leur quantité pourra être facilement déterminé par un homme de métier.

Un édulcorant peut être un sucre naturel comme le saccharose ou le sorbitol ou un produit de synthèse comme la saccharine ou l'aspartame. On utilisera avantageusement la saccharine.

Selon une première forme de réalisation, cette préparation pharmaceutique se présentera sous la forme d'un comprimé effervescent contenant une quantité de composition effervescente telle que définie précédemment correspondant à 200 mg ou 400 mg ou 600 mg d'ibuprofène par comprimé.

Selon une autre forme de réalisation, cette préparation pharmaceutique se présentera sous la forme d'une poudre contenant une quantité de composition effervescente telle que définie précédemment correspondant à 200 mg ou à 400 mg ou 600 mg d'ibuprofène par unité posologique.

Selon un troisième aspect la présente invention a pour objet un procédé de fabrication d'une préparation pharmaceutique sous forme de poudres ou de comprimés effervescents telle que définie précédemment, caractérisé en ce qu'il comprend :
a) le traitement de l'ibuprofène ou l'un de ses sels pharmaceutiquement acceptable par une quantité efficace d'au moins un agent antioxydant pharmaceutiquement acceptable, de préférence par pulvérisation d'une solution ou émulsion contenant l'antioxydant ;
b) le pré-mélange des constituants formant le système effervescent, de préférence sous forme de granulés ;
c) le pré-mélange des autres constituants de la forme pharmaceutique, de préférence sous forme de poudres; et
d) le mélange des produits issus des étapes précédentes a) b) et c).

La forme pharmaceutique obtenue à l'issue du procédé précité pourra être directement utilisée sous forme de poudre et conditionnée par exemple en sachet.

Cette forme pharmaceutique pourra également être présentée en comprimé après une étape de compression traditionnelle.

Dans l'étape a) précitée de traitement de l'ibuprofène, on utilisera une solution ou une émulsion contenant l'agent antioxydant.

Dans le cas de l'alpha-tocophérol qui est hydrophobe, il est avantageux de l'émulsionner dans un solvant approprié comme par exemple de l'eau ou un solvant organique compatible avec l'ibuprofène, c'est-à-dire ne le dissolvant que faiblement.

Divers agents émulsifiants classiquement utilisés en pharmacie peuvent être également utilisés pour réaliser cette émulsion.

Un mélange de docusate de sodium et de polyvinylpyrrolidone s'est avéré particulièrement préféré.

Le traitement de l'ibuprofène par l'agent antioxydant sera réalisé de préférence par pulvérisation de l'émulsion contenant l'antioxydant en utilisant les différentes technologies classiquement utilisées dans l'industrie pharmaceutique comme par exemple un mélangeur planétaire, un mélangeur granulateur sous-vide, un lit fluidisé ou encore un mélangeur à socs, ou une turbine.

Avantageusement, les composants du système effervescent seront soumis à une granulation préalable, ensemble ou de façon séparée, dans un solvant approprié (eau, alcool, dichlorométhane, isopropanol, ou leur mélange).

Les différents ingrédients de la forme pharmaceutique autre que l'ibuprofène traité par l'antioxydant et le système effervescent seront mélangés de préférence sous forme de poudres.

Selon une variante de réalisation, on ajoute au moins un déshydratant interne tel que le citrate de magnesium comme l'un des constituants de la préparation pharmaceutique.

La présente invention sera illustrée par les exemples non limitatifs suivants dans lesquels les constituants sont donnés en parties en poids par rapport à 100 parties en poids d'ibuprofène :

### Exemple 1

### Etape a : Traitement de l'ibuprofène par l'antioxydant

De l'alpha-tocophérol (42.10⁻³), du docusate de sodium (75.10⁻³) et de la polyvinylpyrrolidone (375.10⁻³) sont mélangés dans de l'eau pour obtenir une émulsion.

L'émulsion ainsi obtenue est pulvérisée sur de l'ibuprofène (100) puis le produit ainsi obtenu est séché sous vide.

### Etape b : Granulation

Dans un mélangeur granulateur on introduit les différents composants à granuler, [Bicarbonate de sodium (300) - Acide citrique (500)].

Le liquide de granulation est ensuite pulvérisé sur le lit de poudre.

Après cette phase de granulation, le mélange obtenu est ensuite séché en lit fluidisé.

Les granulés obtenus sont calibrés au moyen d'une grille.

### Etape c : Mélange final

On réalise un mélange des constituants issus des phases a et b avec les autres ingrédients.
- Saccharine sodique : 7,5
- arome : 15
- Lactose : 225
- Citrate de magnésium : 50
- silice colloïdale : 5.10⁻¹

Les produits issus des étapes a à c précitées sont mélangés pendant une durée d'environ trente minutes.

Le produit obtenu se présente sous forme d'une poudre sèche directement utilisable pour la préparation de comprimés.

Par ailleurs, des portions de 3.4 grammes du produit obtenu ont été préssées dans une pastilleuse en permettant ainsi l'obtention de comprimés effervescents.

### Exemple 2

En suivant le mode opératoire décrit ci-dessus, on a préparé des poudres ou des comprimés effervescents ayant la composition suivante :

| Exemples pour comprimés | | | | | |
|---|---|---|---|---|---|
| | **Exemple 2** | **Exemple 3** | **Exemple 4** | **Exemple 5** | **Exemple 6** |
| α Tocophérol | 42.10⁻³ | 84.10⁻³ | 21.10⁻³ | 42.10⁻³ | 42.10⁻³ |
| Docusate de sodium | 75.10⁻³ | 100.10⁻³ | 50.10⁻³ | 80.10⁻³ | 70.10⁻³ |
| PVP | 375.10⁻³ | 350.10⁻³ | 400.10⁻³ | 330.10⁻³ | 420.10⁻³ |
| Ibuprofène | 100 | 100 | 100 | 100 | 100 |
| Bicarbonate de sodium | 585 | 675 | 225 | 270 | 245 |
| Acide citrique | 315 | 305 | 675 | 450 | 285 |
| Saccharide sodique | 10 | 10 | 5 | 5 | 10 |
| Arôme | 20 | 20 | 10 | 5 | 15 |
| Lactose | 820 | - | 520 | 720 | 780 |
| Dextrose | - | - | 100 | - | - |
| Mannitol | - | 600 | - | - | - |
| Citrate de Magnésium | 100 | 100 | 15 | 20 | 20 |
| Benzoate de sodium | - | - | 10 | - | |

| Exemples pour poudre pour sachets | | | | |
|---|---|---|---|---|
| | **Exemple 1** | **Exemple 2** | **Exemple 3** | **Exemple 4** |
| α Tocophérol | 21.10⁻³ | 70.10⁻³ | 30.10⁻³ | 30.10⁻³ |
| Docusate de sodium | 5.10⁻² | 10.10⁻² | 5.10⁻² | 7.10⁻² |
| PVP | 0,5 | 1 | 0.8 | 0,7 |
| Ibuprofène | 100 | 100 | 100 | 100 |
| Bicarbonate de Na | 75 | 160 | 375 | 125 |
| Acide citrique | 225 | 40 | 1125 | 375 |
| Saccharide sodique | 10 | 0 | 5 | 5 |
| Arôme | 0 | 50 | 50 | 50 |
| Lactose | 200 | 4000 | 2000 | 2500 |
| Citrate de Magnésium | 25 | 25 | 25 | 0 |
| Silice colloïdale | - | - | 510⁻¹ | - |

## Revendications

1. Composition pharmaceutique destinée à la préparation de poudres ou de comprimés effervescents contenant une quantité efficace d'ibuprofène ou de l'un de ses sels pharmaceutiquement acceptables à titre d'ingrédient actif ; un système effervescent pharmaceutiquement acceptable comprenant au moins un carbonate alcalin et au moins un acide organique, caractérisée en ce qu'elle comprend au moins un agent antioxydant pharmaceutiquement acceptable, en une quantité suffisante pour stabiliser l'ibuprofène.

2. Composition selon la revendication 1, caractérisée en ce qu'elle comprend en outre une quantité efficace d'au moins un déshydratant interne.

3. Composition selon la revendication 1 ou 2, caractérisée en ce qu'elle comprend en outre un diluant pharmaceutiquement acceptable.

4. Composition selon l'une quelconque des revendications 1 à 3, caractérisée en ce que l'agent antioxydant précité est choisi parmi l'alpha-tocophérol, le gamma-tocophérol, le delta-tocophérol, les extraits d'origine naturelle riches en tocophérol, l'acide L-ascorbique et ses sels de sodium ou de calcium, l'acide palmityl DL ascorbique, le gallate de propyle, le gallate d'octyle, le gallate de dodécyle, le gallate de butyl-hydroxy anisole (BHA) et le gallate de butyl -hydroxy toluène (BHT).

5. Composition selon l'une quelconque des revendications 2 à 4, caractérisée en ce que le déshydratant interne précité est le citrate de magnésium.

6. Composition selon l'une quelconque des revendications 3 à 5, caractérisée en ce que le diluant précité est le lactose.

7. Composition selon l'une quelconque des revendications 1 à 6, pour la préparation de comprimés effervescents, caractérisée en ce qu'elle comprend exprimé en parties en poids pour 100 parties d'ibuprofène, ou de l'un de ses sels pharmaceutiquement acceptables :
- de 120 à 900 et de préférence de 245 à 330 parties en poids d'au moins un carbonate alcalin ;
- de 150 à 1100, et de préférence de 390 à 475 parties en poids d'au moins un acide organique :
- de 21.10⁻³ à 84.10⁻³ d'antioxydant, dans le cas de l'alpha-tocophérol ;
et éventuellement :
- de 400 à 1000, et de préférence de 500 à 800 parties en poids d'au moins un diluant pharmaceutiquement acceptable ; et
- de 10 à 150, et de préférence de 20 à 100 parties en poids d'un déshydratant interne, dans le cas du citrate de magnésium.

8. Composition selon la revendication 7, caractérisée en ce qu'elle comprend exprimé en parties en poids pour 100 parties d'ibuprofène :
- 270 parties en poids d'au moins un carbonate alcalin, de préférence du bicarbonate de sodium ;
- 450 parties en poids d'au moins un acide organique, de préférence l'acide citrique ;
- 42.10⁻³ parties en poids d'alpha-tocophérol ;
- 680 parties en poids d'un diluant, de préférence le lactose;
- 25 parties en poids de citrate de magnésium.

9. Composition selon l'une quelconque des revendications 1 à 6, pour la préparation d'une poudre effervescente, caractérisée en ce qu'elle comprend exprimé en parties en poids pour 100 parties d'ibuprofène ou de l'un de ses sels pharmaceutiquement acceptables :
- de 25 à 1200 et de préférence de 270 à 585 parties en poids d'au moins un carbonate alcalin ;
- de 40 à 1125 , et de préférence de 315 à 675 parties en poids d'au moins un acide organique ;
- de 21.10⁻³ à 84.10⁻³ d'antioxydant, dans le cas de l'alpha-tocophérol; et éventuellement :
- de 90 à 6000 , et de préférence de 200 à 2100 parties en poids d'au moins un diluant pharmaceutiquement acceptable ; et
- de 0 à 100 , et de préférence de 20 à 50 parties en poids d'un déshydratant interne, dans le cas du citrate de magnésium.

10. Composition selon la revendication 9, caractérisée en ce qu'elle comprend exprimé en parties en poids pour 100 parties d'ibuprofène :
- 75 parties d'au moins un carbonate alcalin, de préférence du bicarbonate de sodium ;
- 225 parties en poids d'au moins un acide organique, de préférence l'acide citrique ;
- 42.10⁻³ parties en poids d'alpha-tocophérol ;
- 175 parties en poids d'un diluant, de préférence le lactose;
- 25 parties en poids de citrate de magnésium.

11. Préparation pharmaceutique, sous la forme d'une poudre ou d'un comprimé effervescent, caractérisée en ce qu'elle contient une composition telle que définie à l'une quelconque des revendications 1 à 10, éventuellement associée à au moins un additif usuel choisi parmi les édulcorants, les aromatisants, les colorants et les lubrifiants.

12. Préparation pharmaceutique selon la revendication 11 sous la forme d'un comprimé effervescent, caractérisée en ce qu'elle comprend une quantité de composition effervescente selon l'une quelconque des revendications 1 à 10 correspondant à 200 mg ou 400 mg ou 600 mg d'ibuprofène par comprimé.

13. Préparation pharmaceutique selon la revendication 11 sous la forme d'une poudre effervescente, caractérisée en ce qu'elle contient une quantité de composition effervescente telle que définie à l'une quelconque des revendications 1 à 10 correspondant à 200 mg ou 400 mg 600 mg d'ibuprofène par unité posologique.

14. Procédé de fabrication d'une préparation pharmaceutique sous forme de poudres ou de comprimés effervescents telle que définie à l'une quelconque des revendications 11 à 13, caractérisé en ce qu'il comprend :
a) le traitement de l'ibuprofène ou l'un de ses sels pharmaceutiquement acceptable par une quantité efficace d'au moins un agent antioxydant pharmaceutiquement acceptable, de préférence par pulvérisation d'une solution ou émulsion contenant l'antioxydant ;
b) le pré-mélange des constituants formant le système effervescent, de préférence sous forme de granulés ;
c) le pré-mélange des autres constituants de la forme pharmaceutique, de préférence sous forme de poudres; et
d) le mélange des produits issus des étapes précédentes a) b) et c).

15. Procédé selon la revendication 14, caractérisé en ce que préalablement au traitement de l'ibuprofène ou de l'un de ses sels pharmaceutiquement acceptables, l'agent antioxydant est émulsionné dans un solvant approprié notamment de l'eau, de préférence en présence d'un système émulsifiant comprenant un mélange de docusate de sodium et de polyvinylpyrrolidone.

16. Procédé selon la revendication 14 ou 15, caractérisé en ce qu'on ajoute au moins un déshydratant interne, tel que le citrate de magnésium comme l'un des constituants de la préparation pharmaceutique.

## Claims

1. Pharmaceutical composition for the preparation of effervescent powders or tablets containing an effective amount of ibuprofen or one of its pharmaceutically acceptable salts as the active ingredient; a pharmaceutically acceptable effervescent system comprising at least one alkali metal carbonate and at least one organic acid, characterised in that it comprises at least one pharmaceutically acceptable antioxidant in a sufficient amount to stabilise the ibuprofen.

2. Composition according to claim 1, characterised in that it also comprises an effective amount of at least one internal dehydrating agent.

3. Composition according to claim 1 or 2, characterised in that it also comprises a pharmaceutically acceptable diluent.

4. Composition according to any one of claims 1 to 3, characterised in that the above-mentioned antioxidant is selected from alpha-tocopherol, gamma-tocopherol, delta-tocopherol, extracts of natural origin which are rich in tocopherol, L-ascorbic acid and its sodium or calcium salts, palmityl-DL-ascorbic acid, propyl gallate, octyl gallate, dodecyl gallate, butylhydroxyanisole (BHA) gallate and butylhydroxytoluene (BHT) gallate.

5. Composition according to any one of claims 2 to 4, characterised in that the above-mentioned internal dehydrating agent is magnesium citrate.

6. Composition according to any one of claims 3 to 5, characterised in that the above-mentioned diluent is lactose.

7. Composition according to any one of claims 1 to 6, for the preparation of effervescent tablets, characterised in that it comprises, expressed in parts by weight per 100 parts of ibuprofen or one of its pharmaceutically acceptable salts:
- from 120 to 900 and preferably from 245 to 330 parts by weight of at least one alkali metal carbonate;
- from 150 to 1,100 and preferably from 390 to 475 parts by weight of at least one organic acid;
- from 21.10⁻³ to 84.10⁻³ of antioxidant, in the case of alpha-tocopherol;
and, optionally:
- from 400 to 1,000 and preferably from 500 to 800 parts by weight of at least one pharmaceutically acceptable diluent; and
- from 10 to 150 and preferably from 20 to 100 parts by weight of an internal dehydrating agent, in the case of magnesium citrate.

8. Composition according to claim 7, characterised in that it comprises, expressed in parts by weight per 100 parts of ibuprofen:
- 270 parts by weight of at least one alkali metal carbonate, preferably sodium bicarbonate;
- 450 parts by weight of at least one organic acid, preferably citric acid;
- 42.10⁻³ parts by weight of alpha-tocopherol;
- 680 parts by weight of a diluent, preferably lactose;
- 25 parts by weight of magnesium citrate.

9. Composition according to any one of claims 1 to 6, for the preparation of an effervescent powder, characterised in that it comprises, expressed in parts by weight per 100 parts of ibuprofen or one of its pharmaceutically acceptable salts:
- from 25 to 1,200 and preferably from 270 to 585 parts by weight of at least one alkali metal carbonate;
- from 40 to 1,125 and preferably from 315 to 675 parts by weight of at least one organic acid;
- from 21.10⁻³ to 84.10⁻³ of antioxidant, in the case of alpha-tocopherol; and, optionally:
- from 90 to 6,000 and preferably from 200 to 2,100 parts by weight of at least one pharmaceutically acceptable diluent; and
- from 0 to 100 and preferably from 20 to 50 parts by weight of an internal dehydrating agent, in the case of magnesium citrate.

10. Composition according to claim 9, characterised in that it comprises, expressed in parts by weight per 100 parts of ibuprofen:
- 75 parts of at least one alkali metal carbonate, preferably sodium bicarbonate;
- 225 parts by weight of at least one organic acid, preferably citric acid;
- 42.10⁻³ parts by weight of alpha-tocopherol;
- 175 parts by weight of a diluent, preferably lactose;
- 25 parts by weight of magnesium citrate.

11. Pharmaceutical preparation in the form of an effervescent powder or tablet, characterised in that it contains a composition such as defined in any one of claims 1 to 10, optionally in association with at least one customary additive selected from sweeteners, flavourings, colours and lubricants.

12. Pharmaceutical preparation according to claim 11 in the form of an effervescent tablet, characterised in that it comprises an amount of effervescent composition, according to any one of claims 1 to 10, corresponding to 200 mg, 400 mg or 600 mg of ibuprofen per tablet.

13. A pharmaceutical preparation according to claim 11 in the form of an effervescent powder, characterised in that it contains an amount of effervescent composition, such as defined in any one of claims 1 to 10, corresponding to 200 mg, 400 mg or 600 mg of ibuprofen per dosage unit.

14. Process for the manufacture of a pharmaceutical preparation in the form of effervescent powders or tablets, such as defined in any one of claims 11 to 13, characterised in that it comprises:
a) treating ibuprofen or one of its pharmaceutically acceptable salts with an effective amount of at least one pharmaceutically acceptable antioxidant, preferably by spraying it with a solution or emulsion containing the antioxidant;
b) premixing the constituents forming the effervescent system, preferably in the form of granules;
c) premixing the other constituents of the pharmaceutical form, preferably in the form of powders; and
d) mixing the products resulting from the previous steps a), b) and c).

15. Process according to claim 14, characterised in that prior to the treatment of the ibuprofen or one of its pharmaceutically acceptable salts, the antioxidant is emulsified in an appropriate solvent, especially water, preferably in the presence of an emulsifying system comprising a mixture of sodium docusate and polyvinylpyrrolidone.

16. Process according to claim 14 or 15, characterised in that at least one internal dehydrating agent, such as magnesium citrate, is added as one of the constituents of the pharmaceutical preparation.

## Patentansprüche

1. Pharmazeutische Zusammensetzung zur Herstellung von Brausepulvern oder -tabletten, welche eine wirksame Menge von Ibuprofen oder eines seiner pharmazeutisch annehmbaren Salze als aktiven Bestandteil umfaßt; wobei ein pharmazeutisch annehmbares Brausesystem zumindest ein Alkalicarbonat und zumindest eine organische Säure umfaßt; dadurch gekennzeichnet, daß sie zumindest ein pharmazeutisch annehmbares Antioxidans in einer ausreichenden Menge zur Stabilisation des Ibuprofens enthält.

2. Zusammensetzung nach Anspruch 1, dadurch gekennzeichnet, daß sie außerdem eine wirksame Menge zumindest eines internen Dehydratisierungsmittels umfaßt.

3. Zusammensetzung nach Anspruch 1 oder 2, dadurch gekennzeichnet, daß sie außerdem ein pharmazeutisch annehmbares Verdünnungsmittel umfaßt.

4. Zusammensetzung nach einem der Ansprüche 1 bis 3, dadurch gekennzeichnet, daß das Antioxidans ausgewählt ist aus Alpha-Tocopherol, Gamma-Tocopherol, Delta-Tocopherol, an Tocopherol reichen Extrakten natürlichen Ursprungs, L-Ascorbinsäure und ihren Natrium- oder Calciumsalzen, Palmityl-DL-ascorbinsäure, Propylgallat, Octylgallat, Dodecylgallat, Butylhydroxyanisol (BHA)-gallat und Butylhydroxyltoluol (BHT)-gallat.

5. Zusammensetzung nach einem der Ansprüche 2 bis 4, dadurch gekennzeichnet, daß das interne Dehydratisierungsmittel Magnesiumcitrat ist.

6. Zusammensetzung nach einem der Ansprüche 3 bis 5, dadurch gekennzeichnet, daß das Verdünnungsmittel Lactose ist.

7. Zusammensetzung nach einem der Ansprüche 1 bis 6 zur Herstellung von Brausetabletten, dadurch gekennzeichnet, daß sie, ausgedrückt in Masseteilen pro 100 Teilen Ibuprofen oder eines seiner pharmazeutisch annehmbaren Salze, umfaßt:
- 120 bis 900 und vorzugsweise 245 bis 330 Masseteile zumindest eines Alkalicarbonats;
- 150 bis 1100 und vorzugsweise 390 bis 475 Masseteile zumindest einer organischen Säure;
- 21.10⁻³ bis 84.10⁻³ Antioxidans im Fall von Alpha-Tocopherol;
und gegebenenfalls:
- 400 bis 1000 und vorzugsweise 500 bis 800 Masseteile zumindest eines pharmazeutisch annehmbaren Verdünnungsmittels; und
- 10 bis 150 und vorzugsweise 20 bis 100 Masseteile eines internen Dehydratisierungsmittels im Fall von Magnesiumcitrat.

8. Zusammensetzung nach Anspruch 7, dadurch gekennzeichnet, daß sie, ausgedrückt in Masseteilen pro 100 Teilen Ibuprofen, umfaßt:
- 270 Masseteile zumindest eines Alkalicarbonats, vorzugsweise Natriumbicarbonat;
- 450 Masseteile zumindest einer organischen Säure, vorzugsweise Citronensäure;
- 42.10⁻³ Masseteile Alpha-Tocopherol;
- 680 Masseteile eines Verdünnungsmittels, vorzugsweise Lactose;
- 25 Masseteile Magnesiumcitrat.

9. Zusammensetzung nach einem der Ansprüche 1 bis 6 zur Herstellung eines Brausepulvers, dadurch gekennzeichnet, daß sie, ausgedrückt in Masseteilen pro 100 Teilen Ibuprofen oder eines seiner pharmazeutisch annehmbaren Salze, umfaßt:
- 25 bis 1200 und vorzugsweise 270 bis 585 Masseteile zumindest eines Alkalicarbonats;
- 40 bis 1125 und vorzugsweise 315 bis 675 Masseteile zumindest einer organischen Säure;
- 21.10⁻³ bis 84.10⁻³ Antioxidans im Fall von Alpha-Tocopherol;
und gegebenenfalls:
- 90 bis 6000 und vorzugsweise 200 bis 2100 Masseteile zumindest eines pharmazeutisch annehmbaren Verdünnungsmittels; und
- 0 bis 100 und vorzugsweise 20 bis 50 Masseteile eines internen Dehydratisierungsmittels im Fall von Magnesiumcitrat.

10. Zusammensetzung nach Anspruch 9, dadurch gekennzeichnet, daß sie, ausgedrückt in Masseteilen pro 100 Teilen Ibuprofen, umfaßt:
- 75 Masseteile zumindest eines Alkalicarbonats, vorzugsweise Natriumbicarbonat;
- 225 Masseteile zumindest einer organischen Säure, vorzugsweise Citronensäure;
- 42.10⁻³ Masseteile Alpha-Tocopherol;
- 175 Masseteile eines Verdünnungsmittels, vorzugsweise Lactose;
- 25 Masseteile Magnesiumcitrat.

11. Pharmazeutische Zubereitung in Form eines Brausepulvers oder einer Brausetablette, dadurch gekennzeichnet, daß sie eine wie in einem der Ansprüche 1 bis 10 definierte Zusammensetzung, gegebenenfalls in Vereinigung mit zumindest einem üblichen Additiv, ausgewählt aus Süßstoffen, Aromatisierungsmitteln, Färbemitteln und Gleitmitteln, enthält.

12. Pharmazeutische Zubereitung nach Anspruch 11 in Form einer Brausetablette, dadurch gekennzeichnet, daß sie eine Menge einer Brausezusammensetzung nach einem der Ansprüche 1 bis 10, die 200 mg oder 400 mg oder 600 mg Ibuprofen pro Tablette entspricht, umfaßt.

13. Pharmazeutische Zubereitung nach Anspruch 11 in Form eines Brausepulvers, dadurch gekennzeichnet, daß sie eine Menge einer Brausezusammensetzung, wie in einem der Ansprüche 1 bis 10 definiert, die 200 mg oder 400 mg oder 600 mg Ibuprofen pro Dosierungseinheit entspricht, umfaßt.

14. Verfahren zur Herstellung einer pharmazeutischen Zubereitung in Form von Brausepulvern oder -tabletten, wie in einem der Ansprüche 11 bis 13 definiert, dadurch gekennzeichnet, daß es umfaßt:
a) Behandeln von Ibuprofen oder einem seiner pharmazeutisch annehmbaren Salze mit einer wirksamen Menge zumindest eines pharmazeutisch annehmbaren Antioxidans, vorzugsweise durch Pulverisieren einer das Antioxidans enthaltenden Lösung oder Emulsion;
b) Vormischen der das Brausesystem bildenden Bestandteile, vorzugsweise in Form von Granula;
c) Vormischen der anderen Bestandteile der pharmazeutischen Form, vorzugsweise in Form von Pulvern; und
d) Mischen der aus den vorhergehenden Schritten a), b) und c) erhaltenen Produkte.

15. Verfahren nach Anspruch 14, dadurch gekennzeichnet, daß vor der Behandlung des Ibuprofens oder eines seiner pharmazeutisch annehmbaren Salze das Antioxidans in einem geeigneten Lösungsmittel, insbesondere Wasser, vorzugsweise in Anwesenheit eines Emulgatorsystems, das eine Mischung von Natrium-Docusat und Polyvinylpyrrolidon enthält, emulgiert wird.

16. Verfahren nach Anspruch 14 oder 15, dadurch gekennzeichnet, daß zumindest ein internes Dehydratisierungsmittel, wie Magnesiumcitrat, als einer der Bestandteile der pharmazeutischen Zubereitung zugesetzt wird.
